# FASCICULE DE BREVET EUROPEEN

(11) **EP 2 387 442 B1**
(45) Date de publication et mention de la délivrance du brevet: **02.10.2013**
(21) Numéro de dépôt: 09797110.5
(22) Date de dépôt: 27.11.2009
(51) Int. Cl.: A61Q 19/00, A61K 8/06, A61K 8/73, A61K 8/81, A61K 8/92, A61K 9/107, A61P 17/00

(54) **VÉHICULE SOUS FORME D'UNE ÉMULSION HUILE-DANS-EAU NOTAMMENT DESTINÉ À UNE UTILISATION COSMÉTIQUE OU DERMATOLOGIQUE**
TRÄGER IN FORM EINER ÖL-IN-WASSER-EMULSION, BESONDERS FÜR KOSMETISCHE ODER DERMATOLOGISCHE ZWECKE
CARRIER IN OIL-IN-WATER EMULSION FORM, PARTICULARLY FOR COSMETIC OR DERMATOLOGICAL USE

(30) Priorité: 27.11.2008 FR 0858067
(43) Date de publication de la demande: 23.11.2011
(73) Titulaire: Octalia Technologies, 06220 Vallauris (FR)
(72) Inventeur: SARRAZIN, Christian, F-06580 Pegomas (FR); DO, Marina, F-06500 Menton (FR); BOIX, Michèle, F-06340 Cantaron (FR)
(74) Mandataire: Novagraaf Technologies
(86) Numéro de dépôt international: PCT/FR2009/052323
(87) Numéro de publication internationale: WO 2010/061147

(56) Documents cités:
- EP-A- 1 336 404
- EP-A- 1 598 044
- EP-A- 1 618 864
- EP-A- 1 955 691
- WO-A-96/37180
- WO-A-2008/145943
- US-A- 5 004 598
- US-A- 5 474 979

## Description

La présente invention se rapporte à un véhicule sous forme d'émulsion de type huile-dans-eau (H/E) stable, bien tolérée et non irritante, qui contient une proportion élevée de phase huileuse et une quantité très faible d'agent tensioactif.

La présente invention se rapporte également à un procédé de préparation d'un tel véhicule et à son utilisation comme base pour des compositions cosmétiques ou dermatologiques.

Une émulsion est le mélange de deux liquides non miscibles sous l'effet d'une agitation mécanique formant un système dispersé. Cette dispersion subsiste tant que dure l'agitation mais dès que cette dernière cesse, il y a coalescence des globules et les liquides se séparent.

La formulation d'une émulsion vise à apporter au mélange le ou les produits qui vont permettre ou faciliter la stabilisation du système. Ce ou ces produits sont donc essentiels à la formulation et la conservation en terme de stabilité physico-chimique d'une émulsion ; ils sont appelés émulsifiants, surfactants, émulgateurs agents mouillants ou encore tensioactifs et doivent être incorporés en une quantité relativement importante et généralement reportée en dermatologie et cosmétologie comme pouvant aller jusqu'à 20% en poids du poids total de l'émulsion pour obtenir une stabilité adéquate à la température et dans le temps.

Or les tensioactifs sont connus pour leur caractère irritant, ce qui limite fortement leur utilisation dans les domaines dermocosmétiques.

Ainsi, des effets indésirables liés à l'utilisation de tensioactifs sont observés dans le cadre d'applications dermiques, en particulier des effets allergisants, des irritations cutanées (visage, corps, cuir chevelu), ces effets pouvant se révéler particulièrement gênants pour des peaux sèches et/ou sensibles.

Ce problème est d'autant plus important lorsque l'émulsion est très riche en huile puisqu'il est nécessaire d'augmenter de la même façon la proportion de tensio-actif de façon à stabiliser la composition et éviter son déphasage.

Le problème technique que la présente invention cherche à résoudre consiste donc à fabriquer des émulsions contenant une proportion importante d'huile mais peu de tensio-actifs, de sorte que ces émulsions présentent un compromis satisfaisant entre stabilité de l'émulsion et tolérance tissulaire.

Pour surmonter ce problème technique, l'homme du métier a cherché à supprimer l'utilisation de composés tensio-actifs ou à en réduire les quantités par l'ajout de composés visant à stabiliser l'émulsion tels que des agents viscosants, des charges.

Ainsi par exemple, la demande de brevet européen EP 1 095 659 décrit une préparation sous forme d'une émulsion huile-dans-eau comportant de l'huile, de l'eau et un alcool constituant une phase hydroalcoolique, des principes actifs homéopathiques et/ou végétaux et de l'éther de cellulose, qui est connu de l'homme de l'art pour ses propriétés émulsifiantes. L'éther de cellulose est présent dans le véhicule de EP 1095659 à raison de 0,1% à 10% en poids par rapport au poids total de l'émulsion. Dans un exemple particulier de préparation d'une émulsion de EP 1095659 contenant une proportion de phase huileuse relativement élevée (60% en poids par rapport au poids total de l'émulsion) et constituée de triglycérides à chaîne moyenne, la proportion d'éther de cellulose est de 1% en poids par rapport au poids total du véhicule. Toutefois, les émulsions de EP 1095659 qui sont des émulsions stabilisées par un éther de cellulose, doivent nécessairement contenir de l'alcool, en une proportion pouvant aller de 1 à 50% en poids de la préparation finale. Dans l'exemple particulier cité précédemment, l'alcool est présent à raison d'au moins 8% en poids par rapport au poids total du véhicule.

Les préparations ainsi obtenues présentent certes une bonne stabilité, mais au détriment de la compatibilité tissulaire dans la mesure où la présence d'alcool dans la préparation est connue pour provoquer des irritations tout particulièrement chez les personnes présentant une peau sensible.

La demande de brevet européen EP 1 618 864 décrit également des émulsions riches en huile contenant au moins 60% de phase huileuse en poids par rapport au poids total de la composition et contenant un système émulsionnant en une quantité représentant 2 à 20% en poids du poids total de la composition. Le rapport massique de la quantité de système émulsionnant sur la quantité de phase lipophile varie de 0,04 à 0,2. Afin de renforcer la stabilité physique du système et de contribuer à une texture plus crémeuse, des charges sont ajoutées à raison de 0,5 à 10% en poids du poids total de la préparation.

La demande de brevet internationale WO 96/37180 quant à elle décrit des compositions galéniques utilisables en dermatologie et cosmétologie de type pseudo-émulsions ne faisant pas intervenir de tensio-actifs, mais jouant d'une part sur l'épaississement de la phase aqueuse par l'ajout d'agents gélifiants et d'autre part sur l'épaississement de la phase lipidique par l'emploi d'esters de glycérol. Ces derniers sont des facteurs de consistance semi-solides à température ambiante conférant aux compositions obtenues des structures microscopiques différentes de celles des émulsions. Toutefois, au-delà d'une certaine quantité de phase grasse (supérieure à 20%), les pseudo-émulsions ainsi obtenues perdent en stabilité à la chaleur et/ou dans le temps.

Un but de la présente invention est donc de proposer un véhicule du type précité (émulsion H/E) comportant une proportion élevée de phase huileuse, qui remédie aux inconvénients de l'art antérieur en n'incorporant que de très faibles quantités d'agent tensioactif.

Par proportion élevée de phase huileuse dans une émulsion H/E, on entend, au sens de la présente invention, une proportion d'au moins 10 % en poids de phase huileuse par rapport au poids total de l'émulsion.

Pour atteindre le but de la présente invention, il est proposé un véhicule sous forme d'émulsion huile-dans-eau (H/E) comportant au moins une phase aqueuse (I) et au moins une phase huileuse (II), ladite phase aqueuse (I) comportant un tensioactif hydrosoluble spécifique et au moins un agent viscosant hydrosoluble.

Selon l'invention, la phase huileuse représente au moins 10% en poids du poids total du véhicule et le tensioactif hydrosoluble représente 0,1 à 0,5% en poids, et préférentiellement 0,2 à 0,4% en poids du poids total du véhicule.

Le véhicule selon l'invention est stable, bien toléré et non irritant.

De préférence, la phase huileuse est exempte de tensioactif.

En effet, la faible quantité de tensioactif présent dans la phase aqueuse suffit à assurer la stabilité de l'émulsion et le véhicule ainsi obtenu est bien toléré et non irritant. L'ajout d'un tensioactif supplémentaire dans la phase huileuse n'apporte rien en termes d'amélioration de la stabilité de l'émulsion.

Avantageusement, la phase huileuse représente au plus 60% en poids du poids total du véhicule. En effet, au-dessus de cette valeur, il y a un risque de déphasage de l'émulsion pouvant intervenir quelques jours après la fabrication de l'émulsion, ce risque augmentant, pour une proportion donnée de tensioactifs, lorsque la proportion de phase huileuse augmente au-delà de 60% en poids par rapport au poids total de l'émulsion.

De préférence, la phase huileuse représente environ 15% à 50% en poids du poids total du véhicule. En effet, pour une telle gamme de proportion de phase huileuse, on observe un bon compromis entre une émulsion stable contenant suffisamment de phase huileuse pour une application en cosmétique ou dermo-cosmétique (notamment pour les peaux sèches) et une bonne tolérance tissulaire.

En ce qui concerne la proportion de tensioactifs, au-dessous de 0,1% de tensioactif dans le véhicule selon l'invention, celui-ci présente l'inconvénient de ne pas contenir suffisamment de tensioactif pour permettre d'atteindre une stabilité optimale pour une émulsion contenant une proportion de phase huileuse élevée.

Au-dessus de 0,5% de tensioactif dans le véhicule selon l'invention, celui-ci présente l'inconvénient de contenir une quantité trop importante de tensioactif, et de compromettre la tolérance tissulaire notamment pour les peaux sensibles.

De préférence, le tensioactif hydrosoluble est présent dans le véhicule de l'invention à raison de 0,2 % à 0,4% en poids par rapport au poids total du véhicule. Dans cette gamme préférentielle (0,2 à 0,4% en poids de tensioactif), on obtient une émulsion fine, avec des globules huileux ayant une taille moyenne de l'ordre de 2 µm à 5 µm.

L'agent tensioactif hydrosoluble utilisable dans le véhicule selon l'invention est un alcool polyvinylique (PVA) hydrosoluble, et de manière préférée un alcool polyvinylique partiellement hydrolysé, qui est largement utilisé dans les domaines pharmaceutique et cosmétique.

Par ailleurs, à titre d'agent viscosant hydrosoluble utilisable dans le véhicule selon l'invention, on peut citer les gommes, les glycosaminoglycannes, la cellulose et ses dérivés, les polymères acryliques ou carbomères.

On peut utiliser à titre d'agents viscosants hydrosolubles un xanthane commercialisé par la société CARGILL sous la dénomination commerciale SATIAXANE^{®}, un alginate commercialisé par la société CARGILL sous la dénomination commerciale SATIALGINE^{®}, un acide polyacrylique réticulé commercialisé par la société Lubrizol sous la dénomination commerciale CARBOPOL^{®} 980NF.

La phase huileuse du véhicule selon l'invention est maintenant décrite plus en détail.

La phase huileuse du véhicule selon l'invention peut contenir une ou plusieurs huiles choisies parmi les huiles végétales, les huiles minérales, les huiles siliconées, les huiles de synthèse et les huiles fluorées.

A titre d'huile de synthèse utilisable selon la présente invention, on peut notamment citer le myristate d'isopropyle.

Comme huiles végétales utilisables selon l'invention, on peut citer les huiles de jojoba, avocat, sésame, tournesol, maïs, soja, carthame, pépins de raisin, olive, amande douce, ricin, moringa, coco, palme, bourrache, colza, germe de blé, lin, onagre, argan, calendula et coton.

Comme huiles minérales utilisables selon l'invention, on peut citer les huiles de paraffine, et de préférence les huiles de paraffine liquide.

Un taux de phase grasse important est particulièrement intéressant pour les compositions dermatologiques ou cosmétiques destinées au soin des peaux sèches.

Le véhicule selon l'invention peut en outre comprendre un ou plusieurs additifs compatibles, qui ne modifient pas les caractéristiques propres aux émulsions.

A titre d'additifs compatibles avec un usage cosmétique et/ou dermatologique, on peut citer par exemple les agents conservateurs, les antioxydants, les isotonisants, les agents chélatants, les tampons, les polymères, les charges, les gélifiants hydrophiles ou lipophiles, les filtres hydrophiles, des composés hydrophiles tels que les alcools, les absorbeurs d'odeur, les humectants, et les émollients.

Bien entendu, l'homme de l'art veillera à choisir le ou les éventuels adjuvants à ajouter aux compositions selon l'invention, ainsi que leur concentration, de manière que les propriétés avantageuses attachées intrinsèquement aux compositions conformes à l'invention ne soient pas, ou substantiellement pas altérées par l'addition envisagée.

En particulier, ces adjuvants ne devront pas nuire aux propriétés de la composition selon l'invention, c'est-à-dire une bonne tolérance et l'absence d'irritabilité de la peau.

La présente invention a encore pour objet une composition cosmétique et/ou dermocosmétique et dermatologique comprenant un véhicule selon l'invention, et au moins un actif à usage cosmétique et/ou dermatologique.

L'actif à usage cosmétique et/ou dermatologique peut être hydrosoluble et/ou liposoluble.

Comme actifs à usage dermatologique, on peut citer par exemple les corticoïdes, les antibiotiques, les antifongiques, les antiseptiques, les antiparasitaires, les antiherpétiques, les antiacnéiques, les antiprurigineux, les kératolitiques, les produits pour le traitement du psoriasis, les produits pour le traitement de la dermatite atopique.

Comme actifs hydrosolubles à usage cosmétique et/ou dermocosmétique utilisables selon l'invention, on peut notamment citer les protéines, les acides aminés, les polyols, l'urée, les sucres et les dérivés du sucre, les vitamines hydrosolubles, les extraits végétaux et les hydroxyacides.

Comme actifs liposolubles à usage cosmétique et/ou dermocosmétique utilisables selon l'invention, on peut notamment citer les filtres UVA et UVB, et les vitamines liposolubles telles que le rétinol (vitamine A) et ses dérivés et le tocophérol (vitamine E) et ses dérivés.

La présente invention concerne également un procédé de préparation d'un véhicule, comprenant les étapes assistant à:
a) préparer une première émulsion huile-dans-eau en introduisant dans un réacteur au moins une huile qui représentera 10% à 60% en poids du poids total du véhicule final, puis ajouter une solution aqueuse de tensioactif en une quantité telle que :
   - le rapport massique de la quantité de phase aqueuse dans la première émulsion sur la quantité de phase huileuse dans la première émulsion est compris entre 1/3 et 1/1, et
   - le rapport massique de la quantité de tensio-actif dans le véhicule (c'est-à-dire le véhicule final obtenu à l'issue de l'étape c) sur la quantité d'huile dans le véhicule est compris entre 0,007 et 0,020 ;
b) préparation d'une solution aqueuse d'agent viscosant hydrosoluble, dont la concentration dépend du viscosant utilisé et de l'application visée ;
c) introduction de la première émulsion dans la solution aqueuse d'agent viscosant hydrosoluble, pour obtenir une deuxième émulsion constituant ledit véhicule, la quantité de première émulsion introduite dans la solution aqueuse d'agent viscosant hydrosoluble étant telle que la phase huileuse dans ledit véhicule représente au moins 10% en poids du poids total dudit véhicule et le tensioactif hydrosoluble représente 0,1% à 0,5% en poids du poids total dudit véhicule.

Selon un mode de réalisation avantageux du procédé de l'invention, l'étape a) du procédé de l'invention est réalisée de manière que le rapport massique de la quantité de phase aqueuse dans la première émulsion sur la quantité de phase huileuse dans la première émulsion soit de l'ordre de 1/2. Dans un tel mode de réalisation, la première émulsion obtenue à l'issue de l'étape a) est suffisamment épaisse, mais pas trop, pour pouvoir être facilement manipulée lors de la fabrication du véhicule.

Le tensioactif et l'agent viscosant hydrosolubles sont tels que ceux décrits ci-dessus.

L'invention sera maintenant illustrée à l'aide des exemples non-limitatifs suivants. Les quantités sont indiquées en pourcentage en poids sauf indication contraire.

### EXEMPLES

### Produits

■ Composants de la phase huileuse
   huiles végétales : huile d'amande douce, huile de tournesol, agent tensioactif liposoluble : polyglycéryl-3 dioléate (commercialisé sous la dénomination Plurol Oléique CC497 par la société Gattefossé)
■ Composants de la phase aqueuse
   - eau purifiée
   - agent tensioactif hydrosoluble: alcool polyvinylique (PVA) partiellement hydrolysé commercialisé par la société SIGMA sous la dénomination commerciale MOWIOL® 40-88, agents viscosants hydrosolubles : xanthane commercialisé par la société CARGILL sous la dénomination commerciale SATIAXANE® ; alginate commercialisé par la société CARGILL sous la dénomination commerciale SATIALGINE® ; acide polyacrylique réticulé commercialisé par la société Lubrizol sous la dénomination commerciale CARBOPOL^{®} 980NF.

### EXEMPLE 1

On a préparé un premier exemple de véhicule E1 selon l'invention contenant 25% d'huile en poids du poids total de véhicule et 0,375% de tensio-actif en poids du poids total de véhicule. L'exemple 1 est préparé comme suit.

On réalise tout d'abord une première émulsion (H/E) par dispersion de 30 g d'huile dans 15 g de solution aqueuse de PVA à 3% massique. L'introduction de l'huile dans la solution aqueuse de PVA est faite sous forte agitation (Ultra-turrax pendant 1,5 minute).

Par ailleurs, on prépare 62,5 g de solution aqueuse contenant 1,6% en poids de xanthane.

On introduit ensuite 37,5 g de la première émulsion (H/E) à base de PVA dans la solution aqueuse de xanthane à 1,6% massique, sous agitation au moyen d'une hélice, pour obtenir l'émulsion finale constituant un premier exemple E1 de véhicule selon l'invention. Le poids final est ajusté avec de l'eau.

La composition du véhicule E1 selon l'invention est détaillée dans le tableau 1 ci-après.

### EXEMPLE 2

On a préparé, de la même manière qu'à l'exemple 1, un deuxième exemple E2 de véhicule selon l'invention, contenant 18,8% d'huile en poids du poids total de véhicule et 0,14% de tensio-actif en poids du poids total de véhicule. L'exemple 2 est préparé comme suit.

On réalise tout d'abord une première émulsion (H/E) par dispersion de 22,5 g d'huile dans 22,5 g de solution aqueuse de PVA à 0,75% massique. L'introduction de l'huile dans la solution aqueuse de PVA est faite sous forte agitation (Ultra-turrax pendant 1,5 minute).

Par ailleurs, on prépare 62,5 g de solution aqueuse contenant 1,6% en poids de xanthane.

On introduit ensuite 37,5 g de la première émulsion (H/E) à base de PVA dans la solution aqueuse de xanthane à 1,6% massique, sous agitation au moyen d'une hélice, pour obtenir l'émulsion finale constituant un deuxième exemple E2 de véhicule selon l'invention. Le poids final est ajusté avec de l'eau.

La composition du véhicule E2 selon l'invention est également présentée dans le tableau 1 ci-après.

### EXEMPLE 3

On a préparé, de la même manière qu' à l'exemple 1, un troisième exemple E3 de véhicule selon l'invention contenant 40% d'huile en poids du poids total de véhicule et 0,375% de tensio-actif en poids du poids total de véhicule. L'exemple 3 est préparé comme suit.

On réalise tout d'abord une première émulsion (H/E) par dispersion de 46,6 g d'huile dans 22,4 g de solution aqueuse de PVA à 1,9% massique. L'introduction de l'huile dans la solution aqueuse de PVA est faite sous forte agitation (Ultra-turrax pendant 1,5 minute).

Par ailleurs, on prépare 40 g de solution aqueuse contenant 2,5% en poids de xanthane.

On introduit ensuite 60 g de la première émulsion (H/E) à base de PVA dans la solution aqueuse de xanthane à 2,5% massique, sous agitation au moyen d'une hélice, pour obtenir l'émulsion finale constituant un troisième exemple E3 de véhicule selon l'invention. Le poids final est ajusté avec de l'eau.

La composition du véhicule E3 est présentée dans le tableau 1 ci-après.

### EXEMPLE E4

On a préparé, de la même manière qu'à l'exemple 1, un quatrième exemple E4 de véhicule selon l'invention contenant 18,8% d'huile en poids du poids total de véhicule et 0,375% de tensio-actif en poids du poids total de véhicule et en remplaçant la solution de xanthane par une solution d'alginate. L'exemple 4 est préparé comme suit.

On réalise tout d'abord une première émulsion (H/E) par dispersion de 22,5 g d'huile dans 22,5 g de solution aqueuse de PVA à 2% massique. L'introduction de l'huile dans la solution aqueuse de PVA est faite sous forte agitation (Ultra-turrax pendant 1,5 minute).

Par ailleurs, on prépare 62,5 g de solution aqueuse contenant 3,2% en poids d'alginate.

On introduit ensuite 37,5 g de la première émulsion (H/E) à base de PVA dans la solution aqueuse d'alginate à 3,2% massique, sous agitation au moyen d'une hélice, pour obtenir l'émulsion finale constituant un quatrième exemple E4 de véhicule selon l'invention. Le poids final est ajusté avec de l'eau.

La composition du véhicule E4 est présentée dans le tableau 1 ci-après.

### EXEMPLE 5

On a préparé de la même manière qu' à l'exemple 1, un cinquième exemple E5 contenant 25% d'huile en poids du poids total de véhicule et 0,375% de tensio-actif en poids du poids total de véhicule. L'exemple 5 est préparé comme suit.

On réalise tout d'abord une première émulsion (H/E) par dispersion de 30 g d'huile dans 15 g de solution aqueuse de PVA à 3% massique. L'introduction de l'huile dans la solution aqueuse de PVA est faite sous forte agitation (Ultra-turrax pendant 1,5 minute).

Par ailleurs, on prépare 62,5 g de solution aqueuse contenant 0.48% en poids d'acide polyacrylique réticulé ajusté avec de la soude entre pH 6 et 7. On introduit ensuite 37,5 g de la première émulsion (H/E) à base de PVA dans la solution aqueuse d'acide polyacrylique réticulé à 0,48% massique, sous agitation au moyen d'une hélice, pour obtenir l'émulsion finale constituant un cinquième exemple E5 de véhicule selon l'invention. Le poids final est ajusté avec de l'eau.

La composition du véhicule E5 selon l'invention est détaillée dans le tableau 1 ci-après.

### EXEMPLE COMPARATIF EC1

On a préparé un premier exemple de composition comparative contenant 50% d'huile en poids du poids total de véhicule et 0,375% de tensio-actif en poids du poids total de véhicule.

L'exemple comparatif 1 est préparé comme suit.

On mélange tout d'abord par dispersion 58,3 g d'huile dans 11,7 g de solution aqueuse de PVA à 3,75% massique. L'introduction de l'huile dans la solution aqueuse de PVA est faite sous forte agitation (Ultra-turrax pendant 1,5 minute). On obtient une composition intermédiaire qui n'est pas une émulsion. En effet, dans cette composition intermédiaire, le rapport massique de la quantité de phase aqueuse dans la première émulsion sur la quantité de phase huileuse est de 1/5. Avec un tel rapport massique, on observe un déphasage immédiat de la composition, que l'on n'arrive pas à émulsionner.

Puis, on prépare 40 g de solution aqueuse contenant 2,5% en poids de xanthane.

On introduit ensuite 60 g de la composition intermédiaire à base de PVA dans la solution aqueuse de xanthane à 2,5% massique, sous agitation au moyen d'une hélice, pour obtenir un premier exemple comparatif EC1 de véhicule, dont le poids final est ajusté avec de l'eau. De même que la composition intermédiaire, le véhicule EC1 n'est pas une émulsion.

La composition du véhicule EC1 est présentée dans le tableau 1 ci-après.

### EXEMPLE COMPARATIF EC2

On a préparé un deuxième exemple de composition comparative contenant 25% d'huile en poids du poids total de véhicule et 0,05% de tensio-actif en poids du poids total de véhicule et dans lequel le rapport massique de la quantité de tensio-actif dans le véhicule final sur la quantité d'huile dans le véhicule final n'est plus respecté. L'exemple comparatif 2 est préparé comme suit.

On réalise tout d'abord une première émulsion (H/E) par dispersion de 30 g d'huile dans 15 g de solution aqueuse de PVA à 0,4% massique. L'introduction de l'huile dans la solution aqueuse de PVA est faite sous forte agitation (Ultra-turrax pendant 1,5 minute).

Par ailleurs, on prépare 62,5 g de solution aqueuse contenant 1,6% en poids de xanthane.

On introduit ensuite 37,5 g de la première émulsion (H/E) à base de PVA dans la solution aqueuse de xanthane à 1,6% massique, sous agitation au moyen d'une hélice, pour obtenir un deuxième exemple comparatif EC2 de véhicule selon l'invention, dont le poids final est ajusté avec de l'eau. Le véhicule EC2 est une émulsion instable.

La composition du véhicule EC2 est présentée dans le tableau 1 ci-après.

### EXEMPLE COMPARATIF EC3

On a préparé un troisième exemple de composition comparative contenant 25% d'huile en poids du poids total de véhicule, 0,375% de tensio-actif hydrosoluble en poids du poids total de véhicule et 0.5% de tensioactif liposoluble en poids du poids total de véhicule. L'exemple comparatif 3 est préparé comme suit.

On réalise tout d'abord une phase huileuse constituée de 30g d'huile et de 0.61g de Plurol Oléique CC497. On réalise ensuite une première émulsion par dispersion des 30.61g de phase huileuse dans 15 g de solution aqueuse de PVA à 3% massique. L'introduction de la phase huileuse dans la solution aqueuse de PVA est faite sous forte agitation (Ultra-turrax pendant 1,5 minute).

Par ailleurs, on prépare 62,0 g de solution aqueuse contenant 1,6% en poids de xanthane.

On introduit ensuite 38.0 g de la première émulsion (H/E) à base de PVA dans la solution aqueuse de xanthane à 1,6% massique, sous agitation au moyen d'une hélice, pour obtenir un troisième exemple comparatif EC3 de véhicule selon l'invention, dont le poids final est ajusté avec de l'eau. Le véhicule EC3 est une émulsion stable.

La composition du véhicule EC3 est présentée dans le tableau 1 ci-après.

**Tableau 1**

| Composition (en g/pour 100g de véhicule) | E1 (en g/pour 100g de véhicule ) | E2 (en g/pour 100g de véhicule) | E3 (en g/pour 100g de véhicule ) | E4 (en g/pour 100g de véhicule ) | E5 (en g/pour 100g de véhicule) | EC1 (en g/pour 100g de véhicule) | EC2 (en g/pour 100g de véhicule) | EC3 (en g/pour 100g de véhicule) |
|---|---|---|---|---|---|---|---|---|
| - PVA | 0,375 | 0,14 | 0,375 | 0,375 | 0,375 | 0,375 | 0,05 | 0,375 |
| - xanthane | 1 | 1 | 1 | - | - | 1 | 1 | 1 |
| - alginate | - | - | - | 2 | - | - | - | - |
| - acide polyacrylique réticulé ajusté à pH 6-7 | - | - | - | - | 0.3 | - | - | - |
| - huile | 25 | 18,8 | 40 | 18.8 | 25 | 50 | 25 | 25 |
| - polyglycéryl-3 dioléate | - | - | - | - | - | - | - | 0.5 |
| - eau purifiée | q s.100g | q s.100g | q s.100g | q s.100g | q s.100g | q s.100g | q s.100g | q s.100g |

Pour l'ensemble des compositions selon l'invention E1, E2, E3, E4, E5, EC1, EC2 et EC3, les caractéristiques de l'émulsion suivantes ont été évaluées :
- aspect macroscopique,
- mesure de la taille des globules huileux dans le véhicule par granulométrie laser ; la taille exprimée en µm est reportée pour chaque échantillon.

La tenue de l'émulsion à la centrifugation a aussi été évaluée comme test prévisionnel de stabilité. Les échantillons ont été centrifugés pendant 5 minutes à différentes vitesses et examinés visuellement afin de déterminer les modifications physiques telles que le crémage (accumulation de globules lipidiques en surface) ou le déphasage (séparation complète des phases huileuse et aqueuse).

Les résultats obtenus sont présentés dans le tableau 2 ci-après.

**Tableau 2**

| Compositions | Aspect macroscopique | Taille des globules (µm) | Tenue à la centrifugation |
|---|---|---|---|
| E1 | Emulsion très blanche et homogène | 2,9 | + (Pas de modification) |
| E2 | Emulsion très blanche et homogène | 7,8 | + (Pas de modification) |
| E3 | Emulsion épaisse, très blanche et homogène | 3,4 | + (Pas de modification) |
| E4 | Emulsion très blanche et homogène | 2,9 | + (Pas de modification) |
| E5 | Emulsion très blanche et homogène | 3.3 | + (Pas de modification) |
| EC1 | Déphasage immédiat | Mesure non réalisée : lors de l'étape a) du procédé, la composition intermédiaire déphase immédiatement : on ne réussit pas à fabriquer une émulsion intermédiaire | Mesure non réalisée : lors de l'étape a) du procédé, la composition intermédiaire déphase immédiatement : on ne réussit pas à fabriquer une émulsion intermédiaire immédiatement |
| EC2 | Déphasage rapide | Mesure non réalisée : l'émulsion déphase rapidement | Mesure non réalisée : l'émulsion déphase rapidement |
| EC3 | Emulsion jaunâtre | 33.0 | L'émulsion déphase |

Dans les conditions de fabrication selon l'invention, les exemples E1 à E5 illustrent des émulsions contenant entre 18,8% et 40% d'huile et une faible quantité de tensio-actif comprise entre 0,14% et 0,375%.

La mesure de la taille des globules reflète des tailles caractéristiques généralement admises pour les émulsions classiques contenant largement plus de tensio-actifs (2 à 50 µm).

Par ailleurs, l'évaluation de la stabilité à la centrifugation révèle l'absence de modification physique pour les exemples E1 à E5 selon l'invention alors que ceux-ci contiennent peu de tensioactif.

L'exemple comparatif EC1, dans lequel la proportion de phase aqueuse sur la phase huileuse dans la composition intermédiaire est en-dehors des gammes revendiquées (étape a) du procédé), montre qu'il n'est alors pas possible d'obtenir une émulsion intermédiaire puisqu'on observe un déphasage immédiat.

L'exemple comparatif EC2, dans lequel les proportions de phase huileuse et de tensio-actif dans le véhicule final sont en dehors des gammes revendiquées mais pour lequel on est passé par une émulsion intermédiaire conforme à l'étape a) du procédé de l'invention, montre que le véhicule final obtenu présente un déphasage rapide, témoignant de son instabilité.

L'exemple comparatif EC3, dans lequel on a incorporé un tensioactif liposoluble dans la phase huileuse en plus du tensioactif hydrosoluble (PVA) dans la phase aqueuse montre, comparativement à l'exemple E1, que l'émulsion finale EC3, bien que techniquement réalisable, déphase lors du test de rupture à la centrifugation, ce qui témoigne de son instabilité. Elle présente par ailleurs une taille de globules plus importante que dans l'exemple E1.

## Revendications

1. Véhicule sous forme d'émulsion huile-dans-eau (H/E) comportant au moins une phase aqueuse et au moins une phase huileuse, cette phase huileuse représentant au moins 10% en poids du poids total du véhicule, ladite phase aqueuse comportant un tensioactif hydrosoluble représentant 0,1% à 0,5% en poids du poids total du véhicule et au moins un agent viscosant hydrosoluble, **caractérisé en ce que** l'agent tensioactif hydrosoluble est un alcool polyvinylique (PVA) hydrosoluble.

2. Véhicule selon la revendication 1, **caractérisé en ce que** l'alcool polyvinylique (PVA) hydrosoluble est partiellement hydrolysé.

3. Véhicule selon l'une quelconque des revendications 1 et 2, **caractérisé en ce que** la phase huileuse est exempte de tensioactif.

4. Véhicule selon l'une quelconque des revendications 1 à 3, **caractérisé en ce que** le tensioactif hydrosoluble représente 0,2% à 0,4% en poids du poids total du véhicule.

5. Véhicule selon l'une quelconque des revendications 1 à 4, **caractérisé en ce que** la phase huileuse représente au plus 60% en poids du poids total du véhicule.

6. Véhicule selon la revendication 5, **caractérisé en ce que** la phase huileuse représente environ 15% à 50% en poids du poids total du véhicule.

7. Véhicule selon l'une quelconque des revendications précédentes, **caractérisé en ce que** l'agent viscosant hydrosoluble est choisi parmi les gommes, les glycosaminoglycannes, la cellulose et ses dérivés, les polymères acryliques ou carbomères.

8. Véhicule selon l'une quelconque des revendications précédentes, **caractérisé en ce que** la phase huileuse comprend au moins une huile choisie dans le groupe constitué des huiles minérales, des huiles végétales, des huiles siliconées, des huiles de synthèse et des huiles fluorées.

9. Véhicule selon la revendication 8, **caractérisé en ce que** l'huile est une huile végétale choisie parmi les huiles de jojoba, avocat, sésame, tournesol, maïs, soja, carthame, pépins de raisin, olive, amande douce, ricin, moringa, coco, palme, bourrache, colza, germe de blé, lin, onagre, argan, calendula et coton.

10. Véhicule selon l'une quelconque des revendications précédentes, **caractérisé en ce qu'**il comprend un ou plusieurs additifs choisi(s) parmi les agents conservateurs, les antioxydants, les isotonisants, les agents chélatants, les tampons, les polymères, les charges, les gélifiants hydrophiles ou lipophiles, des composés hydrophiles tels que les alcools, les filtres hydrophiles, les absorbeurs d'odeur, les humectants et les émollients.

11. Composition cosmétique et/ou dermatologique se présentant sous forme d'une émulsion huile-dans-eau, **caractérisée en ce qu'**elle comprend :
- un véhicule tel que défini selon l'une quelconque des revendications 1 à 10, et
- au moins un actif à usage cosmétique et/ou dermatologique.

12. Composition selon la revendication 11, **caractérisée en ce que** l'actif est un actif à usage dermatologique choisi parmi les corticoïdes, les antibiotiques, les antifongiques, les antiseptiques, les antiparasitaires, les antiherpétiques, les antiacnéiques, les antiprurigineux, les kératolitiques, les produits pour le traitement du psoriasis, les produits pour le traitement de la dermatite atopique.

13. Composition selon la revendication 11, **caractérisée en ce que** l'actif est un actif cosmétique et/ou dermocosmétique choisi parmi les protéines, les acides aminés, les polyols, l'urée, les sucres et les dérivés du sucre, les vitamines hydrosolubles, les extraits végétaux et les hydroxyacides.

14. Procédé de préparation d'un véhicule tel que défini selon l'une quelconque des revendications 1 à 10, comprenant les étapes successives suivantes :
a) préparer une première émulsion huile-dans-eau en introduisant dans un réacteur au moins une huile qui représentera 10% à 60% en poids du poids total du véhicule final, puis ajouter une solution aqueuse de tensioactif en une quantité telle que :
- le rapport massique de la quantité de phase aqueuse dans la première émulsion sur la quantité de phase huileuse dans la première émulsion est compris entre 1/3 et 1/1,
- le rapport massique de la quantité de tensio-actif dans ledit véhicule sur la quantité d'huile dans ledit véhicule est compris entre 0,007 et 0,020,
b) préparation d'une solution aqueuse d'agent viscosant hydrosoluble,
c) introduction de la première émulsion dans la solution aqueuse d'agent viscosant hydrosoluble, pour obtenir une deuxième émulsion constituant ledit véhicule, la quantité de première émulsion introduite dans la solution aqueuse d'agent viscosant hydrosoluble étant telle que la phase huileuse dans ledit véhicule représente au moins 10% en poids du poids total dudit véhicule et le tensioactif hydrosoluble représente 0,1% à 0,5% en poids du poids total dudit véhicule.

15. Procédé selon la revendication 14, **caractérisé en ce que** le rapport massique de la quantité de phase aqueuse dans la première émulsion sur la quantité de phase huileuse dans la première émulsion est de 1/2.

## Patentansprüche

1. Träger in Form einer Öl-in-Wasser-Emulsion (O/W-Emulsion), die zumindest eine wässrige Phase und zumindest eine ölige Phase umfasst, wobei diese ölige Phase zumindest 10 Gewichtsprozent des Gesamtgewichts des Trägers darstellt, wobei die besagte wässrige Phase ein wasserlösliches Tensid umfasst, das 0,1 bis 0,5 Gewichtsprozent des Gesamtgewichts des Trägers darstellt, und zumindest ein wasserlösliches viskosierendes Mittel, **dadurch gekennzeichnet, dass** das wasserlösliche Tensid ein wasserlöslicher Polyvinylalkohol (PVA) ist.

2. Träger nach Anspruch 1, **dadurch gekennzeichnet, dass** der wasserlösliche Polyvinylalkohol (PVA) teilweise hydrolisiert ist.

3. Träger nach einem beliebigen der vorstehenden Ansprüche 1 und 2, **dadurch gekennzeichnet, dass** die ölige Phase frei von Tensid ist.

4. Träger nach einem beliebigen der vorstehenden Ansprüche 1 bis 3, **dadurch gekennzeichnet, dass** das wasserlösliche Tensid 0,2 bis 0,4 Gewichtsprozent des Gesamtgewichts des Trägers darstellt.

5. Träger nach einem beliebigen der vorstehenden Ansprüche 1 bis 4, **dadurch gekennzeichnet, dass** die ölige Phase höchstens 60 Gewichtsprozent des Gesamtgewichts des Trägers darstellt.

6. Träger nach Anspruch 5, **dadurch gekennzeichnet, dass** die ölige Phase etwa 15 bis 50 Gewichtsprozent des Gesamtgewichts des Trägers darstellt.

7. Träger nach einem beliebigen der vorstehenden Ansprüche, **dadurch gekennzeichnet, dass** das wasserlösliche viskosierende Mittel unter den Gummis, den Glykosaminoglykanen, der Zellulose und deren Derivate, den Acrylpolymeren oder Karbomeren gewählt wird.

8. Träger nach einem beliebigen der vorstehenden Ansprüche, **dadurch gekennzeichnet, dass** die ölige Phase zumindest ein Öl umfasst, das in der aus Mineralölen, Pflanzenölen, Silikonölen, Syntheseölen und fluorierten Ölen gebildeten Gruppe gewählt wird.

9. Träger nach Anspruch 8, **dadurch gekennzeichnet, dass** das Öl ein Pflanzenöl ist, das unter den Jojoba-, Avocado-, Sesam-, Sonnenblumen-, Mais-, Soja-, Färberdistel-, Traubenkern-, Oliven-, Süßmandel-, Rizinus-, Moringa-, Kokos-, Palm-, Borretsch-, Raps-, Weizenkeim-, Lein-, Nachtkerzen-, Argan-, Ringelblumen- und Baumwollölen gewählt wird.

10. Träger nach einem beliebigen der vorstehenden Ansprüche, **dadurch gekennzeichnet, dass** er einen oder mehrere Zusatzstoffe umfasst, der (die) unter den Konservierungsmitteln, Antioxidantien, Isotonisierungsmitteln, Chelatliganden, Puffern, Polymeren, Füllstoffen, wasser- oder fettabweisenden Gelatinierungsmitteln, wasserabweisenden Verbindungen wie Alkohol, wasserabweisenden Filtern, Geruchsabsorbern, Benetzungsmitteln, Erweichungsmitteln gewählt wird (werden).

11. Kosmetische und/oder dermatologische Zusammensetzung in Form einer Wasser-in-Öl-Emulsion, **dadurch gekennzeichnet, dass** sie umfasst:
- Einen Träger, wie er nach einem beliebigen der Ansprüche 1 bis 10 definiert ist,
- Mindestens einen Aktivstoff für den kosmetischen und/oder dermatologischen Gebrauch.

12. Zusammensetzung nach Anspruch 11, **dadurch gekennzeichnet, dass** der Aktivstoff ein solcher für den dermatologischen Gebrauch ist, der unter den Kortikoiden, Antibiotika, Fungiziden, Antiseptika, Antiparasitika, Antiherpetika, Anti-Akne-Mitteln, Antipruritika, Keratolitika, Produkten für die Behandlung der Psoriasis, Produkten für die Behandlung der atopischen Dermatitis gewählt wird.

13. Zusammensetzung nach Anspruch 11, **dadurch gekennzeichnet, dass** der Aktivstoff ein kosmetischer und/oder dermokosmetischer Aktivstoff ist, der unter den Proteinen, Aminosäuren, Polyolen, Harnstoff, Zuckern und Zuckerderivaten, wasserlöslichen Vitaminen, Pflanzenextrakten und Hydroxysäuren gewählt wird.

14. Verfahren für die Zubereitung eines Trägers wie er nach einem beliebigen der Ansprüche 1 bis 10 definiert ist, das nacheinander die folgenden Schritte umfasst:
a) Eine erste Öl-in-Wasser-Emulsion zubereiten, indem zumindest ein Öl in einen Reaktor eingegeben wird, das 10 bis 60 Gewichtsprozent des Gesamtgewichts des endgültigen Trägers darstellen wird, dann eine wässrige Tensidlösung in einer solchen Menge eingeben, dass:
- das Massenverhältnis der Menge der wässrigen Phase in der ersten Emulsion zur Menge der öligen Phase in der ersten Emulsion bei zwischen 1/3 und 1/1 liegt,
- das Massenverhältnis der Tensidmenge im besagten Träger zur Ölmenge im besagten Träger bei zwischen 0,007 und 0,020 liegt,
b) Zubereitung einer wässrigen Lösung aus wasserlöslichem viskosierendem Mittel,
c) Eingabe der ersten Emulsion in die wässrige Lösung aus wasserlöslichem viskosierendem Mittel, um eine zweite Emulsion zu erzielen, die den besagten Träger bildet, wobei die Menge der in die wässrige Lösung aus wasserlöslichem viskosierendem Mittel eingegebenen Emulsion dergestalt ist, dass die ölige Phase im besagten Träger zumindest 10 Gewichtsprozent des Gesamtgewichts des besagten Trägers darstellt und das wasserlösliche Tensid 0,1 bis 0,5 Gewichtsprozent des Gesamtgewichts des besagten Trägers darstellt.

15. Verfahren nach Anspruch 14, **dadurch gekennzeichnet, dass** das Massenverhältnis der Menge wässriger Phase in der ersten Emulsion zur Menge öliger Phase in der ersten Emulsion bei 1/2 liegt.

## Claims

1. A vehicle in the form of an oil-in-water (OIW) emulsion comprising at least an aqueous phase and at least an oily phase, this oily phase representing at least 10% in weight of the total weight of the vehicle, said aqueous phase comprising a water-soluble surfactant representing 0.1% to 0.5% in weight of the total weight of the vehicle and at least a water-soluble viscosing agent, **characterized in that** the water soluble surfactant is a water-soluble polyvinyl alcohol (PVA).

2. The vehicle according to claim 1, **characterized in that** the water-soluble polyvinyl alcohol (PVA) is partially hydrolyzed.

3. The vehicle according to any one of claims 1 and 2, **characterized in that** the oily phase is surfactant free.

4. The vehicle according to any one of claims 1 to 3, **characterized in that** the water-soluble surfactant represents 0.2% to 0.4% in weight of the total weight of the vehicle.

5. The vehicle according to any one of claims 1 to 4, **characterized in that** the oily phase represents 60% at the most in weight of the total weight of the vehicle.

6. The vehicle according to claim 5 **characterized in that** the oily phase represents about 15% to 50% in weight of the total weight of the vehicle.

7. The vehicle according to any one of the preceding claims, **characterized in that** the water-soluble viscosing agent is selected from among gums, glycosaminoglycans, cellulose and its derivatives, acrylic polymers or carbomers.

8. The vehicle according to any one of the preceding claims, **characterized in that** the oily phase comprises at least one oil selected from among the group constituted of mineral oils, vegetable oils, silicon oils, synthetic oils and fluorinated oils.

9. The vehicle according to claim 8, **characterized in that** the oil is a vegetable oil selected from among jojoba, avocado, sesame, sunflower, corn, soya, safflower, grapeseed, olive, sweet almond, castor, moringa, coconut, palm, borage, rapeseed, wheatgerm, linseed, evening primrose, argan, calendula and cottonseed oils.

10. The vehicle according to any one of the preceding claims, **characterized in that** it comprises one or several additives selected from among preservatives, antioxidants, isotonizing agents, chelating agents, buffers, polymers, fillers, hydrophilic or lipophilic gelling agents, hydrophilic compounds such as alcohols, hydrophilic filters, odor absorbents, humectants and emollients.

11. A cosmetic and/or dermatological composition in the form of an oil-in-water emulsion, **characterized in that** it comprises:
- a vehicle such as defined according to any one of claims 1 to 10, and
- at least an active for cosmetic and/or dermatological use.

12. The composition according to claim 11, **characterized in that** the active is an active for dermatological use selected from among corticoids, antibiotics, antifungal agents, antiseptics, antiparasitics, anti-herpes agents, anti-acne agents, anti-pruritic agents, keratolitics, products for treating psoriasis, products for treating atopic dermatitis.

13. The composition according to claim 11, **characterized in that** the active is a cosmetic and/or dermo-cosmetic active selected from among proteins, amino acids, polyols, urea, sugars and sugar derivatives, water-soluble vitamins, vegetable extracts and hydroxyl acids.

14. A method for preparing a vehicle such as defined according to any one of claims 1 to 10, comprising the following successive steps:
a) preparing a first oil-in-water emulsion by introducing into a reactor at least one oil which will represent 10% to 60% in weight of the total weight of the final vehicle, then adding an aqueous surfactant solution in a quantity such that:
- the mass ratio of the quantity of aqueous phase in the first emulsion on the quantity of oily phase in the first emulsion is comprised between 1/3 and 1/1.
- the mass ratio of the quantity of surfactant in said vehicle on the quantity of oil in said vehicle is comprised between 0.007 and 0.020,
b) preparing an aqueous solution of water-soluble viscosing agent,
c) introducing the first emulsion into the aqueous solution of water-soluble viscosing agent, to obtain a second emulsion constituting said vehicle, the quantity of first emulsion introduced into the aqueous solution of water-soluble viscosing agent being such that the oily phase in said vehicle represents at least 10% in weight of the total weight of said vehicle and the water-soluble surfactant represents 0.1% to 0.5% in weight of the total weight of said vehicle.

15. The method according to claim 14, **characterized in that** the mass ratio of the quantity of aqueous phase in the first emulsion on the quantity of oily phase in the first emulsion is of 1/2.
